(19) Europäisches Patentamt
European Patent Office
Office européen des brevets

(11) Publication number: 0 234 475
A2

# (12) EUROPEAN PATENT APPLICATION

(21) Application number: 87102183.8

(22) Date of filing: 16.02.87

(51) Int. Cl.⁴: A61K 7/075

(30) Priority: 24.02.86 US 832566

(43) Date of publication of application:
02.09.87 Bulletin 87/36

(84) Designated Contracting States:
AT BE CH DE ES FR GB GR IT LI LU NL SE

(71) Applicant: S.C. JOHNSON & SON, INC.
1525 Howe Street
Racine, Wisconsin 53403(US)

(72) Inventor: Padden, Timothy J.
2616 Oregon Street
Racine Wisconsin 53406(US)
Inventor: Tait, W.Stephen
2048 St. Clar Street
Racine Wisconsin 53402(US)

(74) Representative: Baillie, Iain Cameron et al
c/o Ladas & Parry Isartorplatz 5
D-8000 München 2(DE)

## (54) Non-corrosive hair conditioning foam compositions.

(57) Hair conditioning foam compositions containing a quaternary ammonium phosphate compound as both the conditioning agent and the foaming agent are substantially non-corrosive to organic polymer lined tin-plate steel cans.

EP 0 234 475 A2

The present invention relates to a hair conditioning foam composition which is non-corrosive to organic polymer lined tin-plate steel cans.

Hair conditioning foams, commonly referred to as mousses, are employed to deliver holding and conditioning agents to hair. Hair conditioning foams can be applied to wet or dry hair and need not be rinsed from the hair after application. Typically, quaternary ammonium compounds, and in particular quaternary ammonium chlorides and sulfates, are employed in hair conditioning foams as the conditioning agent.

U. S. Patent 4, 536,390 discloses a collapsible foam aerosol hair product. The product disclosed therein contains a polymeric setting agent, a quaternary ammonium compound that acts as a conditioning agent and a foaming agent, water and a propellent. Other ingredients are optionally added such as, for example, preservatives, perfumes, alcohol, and corrosion inhibitors. U.S. Patent 4,536,390 identifies the corrosion problems exhibited by such aerosol foam hair products. Because of this corrosion problem it is preferred in that patent to utilize lined aluminum containers for such products. Aluminum cans are not the container of choice for hair conditioning products due to cost and lack of availability in many locations. Additionally, aluminum cans are not magnetic and because of this require more costly handling procedures than required for lined tin-plate steel cans which are magnetic. hydroxyethyl cetyldimonium phosphate is a known cationic hair conditioning agent especially useful in oxidative treatments from cold permanent wave neutralizers and pre-rinses. It also imparts excellent holding properties, volume, and conditioning effects to styling mousses. For use in styling mousses, it is recommended to use hydroxyethyl cetyldimonium phosphate in combination with ethanol, a foaming agent, a propellent and water. The recommended use level of hydroxyethyl cetyldimonium phosphate is from 0.1 to 0.5% by weight. See Technical Bulletin COS-6 for BINA Qat 44C brand hydroxyethyl cetyldimonium phosphate commercially available from Dyestuffs and Chemical Division, Ciba-Geigy Corporation, P.O. Box 18300, Greensboro, NC 27419.

It is, therefore, the object of the present invention to produce a hair conditioning foam composition which is substantially non-corrosive to organic polymer lined tin-plate steel cans.

The present invention provides a hair conditioning foam composition containing a polymeric setting and foam strengthening agent, a quaternary ammonium conditioning and foaming agent, water and a propellent, characterized by the quaternary ammonium conditioning and foaming agent being a quaternary ammonium phosphate in an amount effective to maintain the integrity of an organic polymer lining of a lined tin-plate steel can containing said composition thereby substantially preventing corrosion of the lined tin-plate steel can.

The present invention also provides a method of protecting an organic polymer lined tinplate can from corrosion by a hair conditioning foam composition, characterized by employing as a conditioning and foaming agent for the hair conditioning foam composition a quaternary ammonium phosphate compound in an amount effective to substantially pre vent corrosion of the organic polymer lined tin-plate can when the composition is stored in the tin-plate can.

Briefly, in accordance with the present invention, a substantially non-corrosive hair conditioning aerosol foam composition is prepared by employing a quaternary ammonium phosphate compound as both the conditioning agent and the foaming agent. This hair conditioning aerosol foam is non-corrosive to tin-plate steel cans which are lined with an organic polymer. The use of tin-plate steel cans provides cost and handling advantages and such cans are generally more available than aluminum cans.

In the preferred practice of the present invention, hydroxyethyl cetyldimonium phosphate is employed as both a conditioning agent and a foaming agent in a hair conditioning aerosol foam which is suitable for packaging in organic polymer lined tin-plate steel cans. The hydroxyethyl cetyldimonium phosphate is present in the foam composition in concentrations of 0.60 -5.0% by weight. The foam composition additionally contains a polymeric setting agent, a propellent and water. Optionally, other cosmetic additives are employed in the present foam compositions.

The invention will now be described in detail.

In practicing the present invention, a quaternary ammonium compound is employed as both the conditioning and foaming agent in a hair conditioning foam composition. The quaternary ammonium compound substantially prevents corrosion of an organic polymer lined tin-plate steel can containing the hair conditioning foam composition. The quaternary ammonium compounds can be any quaternary ammonium phosphate which has both hair conditioning and foaming properties. Preferred quaternary ammonium phos phates are those compounds of the formula:

$$\left[ CH_3(CH_2)_x-\underset{\underset{R^1}{|}}{\overset{\overset{R}{|}}{N}}-CH_2\ CH_2\ OH \right]^+ \left[ H_2PO_4 \right]^-$$

wherein x represents 8 to 22, inclusive and R and R' each independently represent an alkyl group having up to 22 carbon atoms. Preferably R and R' represent a lower alkyl ($C^1$-$C^4$) such as methyl. Mixtures of these compounds can also be employed. An especially preferred quaternary ammonium phosphate is hydroxyethyl cetyldimonium phosphate (BINA QAT 44C).

The quaternary ammonium conditioning and foaming agent is added to the present hair conditioning foam compositions in amounts which substantially prevent corrosion of an organic polymer lined tin-plate steel can containing such a hair conditioning foam composition. Generally, the quaternary ammonium compound is added to the foam composition in amounts of from about 0.60 to about 5.0% by total weight of the foam composition and preferably from about 0.75 to about 3.0% by weight of the total foam composition. It is especially preferred to add the quaternary ammonium compounds to the present hair conditioning foam compositions in an amount of from about 0.75% to about 1.0% by weight of the foam composition.

The quaternary ammonium conditioning and foaming agent employed in the practice of the present invention replaces the standard quaternary ammonium chloride conditioning agents and/or the quaternary ammonium sulfate conditioning agents. Small amounts (less than 0.5%) of these chloride and sulfate quaternium conditioning agents may be retained in the formulations while not substantially affecting the the corrosion of the organic polymer lined tinplate steel cans.

The other components of the present hair conditioning foam compositions are standard hair conditioning compositions and include polymeric setting agents, propellents, water and other cosmetic additives. All of these components are well known to one skilled int he art and are employed in concentrations readily determinable by one skilled in the art.

Suitable as polymeric setting agents include high molecular weight polymers that are water soluble and stable in an environment having a pH of less than about 7. Suitable polymers include Onamer M polymer, Mirapol A-15 polymer, Celquat H-100 and Celquat L-200 polymers which are copolymers of hydroxyethyl cellulose and diallyl ammonium chloride, copolymers of acrylamide and betamethacryl oxyethyl methylamine ammonium methyl sulfate (Catamer Q and Reten brand polymers), the polymers dimethyl diallyl ammonium chloride, the polymeric quaternary ammonium salts of acrylamide and dimethyl diallyl ammonium chloride (Merquat 550), the polymeric quaternary ammonium salts of methyl and stearyl dimethyl amino ethyl methacrylate quaternized with dimethyl sulfate, polydimethylaminoethyl methacrylate quaternized with methyl bromide, the quaternary ammonium polymer formed by the reaction of dimethyl sulfate and a copolymer of vinyl pyrrolidone and dimethyl aminoethylmethacrylate (GAFQUAT 734 and 755), the polymeric quaternary ammonium salt prepared by the reaction of ethylmethacrylate, abietyl methacrylate diethylaminoethyl methacrylate copolymer quaternized with dimethyl sulfate, and polymers prepared from vinyl pyrrolidone and dimethyl aminoethylmethacrylate monomers (Copolymer 845, Copolymer 937 and Copolymer 958 available from GAF). Polyvinylpyrrolidone (PVP) and mixtures of PVP and vinyl acetate can also be used as setting agents.

The above polymeric setting agents are added to the present foam compositions in amounts of from about 0.1 to about 5.0% by total weight of the composition. The optimum amount employed in the present foam compositions is readily determinable by one skilled in the art and will depend upon a variety of factors, such as, for example, the particular polymeric setting agent employed, the specific purpose of the foaming composition, and the particular hair conditions in which the foam composition is formulated for.

The present foam compositions contain a substantial amount of propellent. Generally, the propellent can be present in amounts of from about 10 to about 90% by total weight of the foam composition advantageously from about 30 to about 70% by total weight of the foam composition and preferably from about 30 to about 50% by total weight of the composition. Suitable as propellents are any of the standard liquified propellent materials commonly used in aerosol formulations. These include the hydrocarbon propellents such as propane, butane, iosbutane, etc. Mixtures of hydrocarbon propellents can also be employed. Preferred propellents include A-46 propellent which is a mixture of propane and isobutane in a weight ratio of 15.4/84.6 and B-52 propellent which is a mixture of propane/isobutane/n-butane in a weight ratio of 26.4/26.5/47.1.

The balance of the present hair conditioning foam composition is water and other conventional cosmetic additives. Preferably deionized water is employed in the present formulations. Additional cosmetic additives include preservatives, dyes and perfumes.

In a preferred embodiment of the present in vention, hydroxyethyl cetyldimonium phosphate is employed as the quaternary ammonium phosphate compound. Hydroxyethyl cetyldimonium phosphate is commercially available as BINA QAT 44C from Dyestuffs and Chemical Division of Ciba-Geigy, P. O. Box 18300, Greensboro, N.C. 27419. Typical hydroxyethyl cetyldimonium phosphate containing formulations according to the present invention include the following:

| | WEIGHT PERCENT | |
|---|---|---|
| INGREDIENTS | Range | Weight |
| Water | 9.3-80 | 51.59 |
| HCP* | 0.6-5.0 | 0.81 |
| Polymeric setting agent | 0.1-10.00 | 4.80 |
| Quaternary ammonium chloride | 0-0.5 | 0.15 |
| Fragrance | 0-2.5 | 0.48 |
| Propellent | 10-90 | 40.0 |

*Hydroxyethyl cetyldimonium phosphate (BINA QAT 44C)

In formulating the present hair conditioning foam compositions an intermediate is prepared by admixing the polymeric setting agent, the quaternary ammonium phosphate, water and any other additional cosmetic additives. The propellent and the intermediate composition are then admixed during the packaging operation into an organic polymer lined tin-plate aerosol container. These aerosol containers are fitted with standard dip tube and valving such that a foam will be produced upon actuation of the valve. Dip tubes are not required on containers which are actuated by inversion. Since the intermediate and the propellent are not always completely compatible over long periods of time, a microdip tube is preferred since this minimizes the amount of free propellent which might float on top of the aqueous intermediate in the dip tube. Further, some head space should be provided so that the aqueous intermediate and propellent can be redispersed by shaking the container before use.

The organic polymer lined tin-plate steel cans are well known materials routinely used in the aerosol container industry. They are prepared by coating the inside of a tin-plate steel can with an organic polymer employing standard coating procedures. Suitable polymeric coatings include lacquers and epoxies and in particular phenolic modified epoxies, urea modified epoxies and vinyl epoxies. A preferred tin-plate steel can is ASTM Number ANSI-ASTM A 623-77 coated with any of the above-identified lining materials. The coated tin-plate cans are packaged employing conventional packaging procedures.

The hair conditioning foam composition of the present invention can be applied to either damp or dry hair. A small amount of the foam composition is dispensed from the container onto the hair or hand of the user. The foam is then distributed uniformly throughout the hair by massaging, combing or brushing. The hair is then styled or set as usual.

The following examples illustrate the practice of the present invention but should not limit its scope. All percentages are by weight of active ingredient unless specified otherwise. Below is a description of the ingredients used in the Examples:

| INGREDIENT | DESCRIPTION |
|---|---|
| KATHON CG | methylchloroisothiazolinone and methylisothiazolinone (1.5% by weight) |
| BINA QAT 44C | hydroxyethyl cetyldimonium phosphate (30% by weight) |

| INGREDIENTS (Continued): | DESCRIPTION (Con't): |
|---|---|
| KEMAMINE BQ-2982-B | erucyl dimethyl benzyl ammonium chloride (50% by weight) |
| Copolymer 845 | vinyl pyrrolidone/dimethyl aminoethylmethacrylate co-polymer (20% by weight) |
| Fragrance 509782 | Fritzsche D+O fragrance |
| A-46 propellent | propane (15.44%)/isobutane (84.6%) |
| B-52 propellent | propane (26.4%)/isobutane (26.5%/n-butane (47.1%) |

EXAMPLE I: Preparation of Intermediate

The following intermediate was prepared by adding the ingredients to the deionized water in the order given with mixing in between each addition:

INTERMEDIATE A

| INGREDIENTS | WEIGHT PERCENT |
|---|---|
| Deionized water | 95.8 |
| 5% KATHON CG* | 0.2 |
| HCP** | 1.35 |
| KEMAMINE BQ 2982-B | 0.25 |
| Copolymer 845 | 1.6 |
| Fragrance 509782 | 0.8 |

pH = 4.3

*5% KATHON CG represents 5 parts by weight KATHON CG (1.5% active) and 95 parts by weight deionized water.

**Hydroxyethyl cetyldimonium phosphate (BINA QAT 44C)

Intermediate A was then formulated into Formulation A by filling into organic polymer lined tin-plate steel cans with 68.01 milliliters (ml) of Intermediate A and 81.79 ml (45.37g) of B-52 propellent. The cans were fitted with an Iris foam spout and a Precision one inch aluminum valve with a 2 × 0.020" stem and a microdip tube. The cans were pressurized after applying a 22" vacuum and then hot tanked at 130°F. The cans were maintained at this temperature for one hour after filling.

EXAMPLE 2

Substantially the same procedures described in Example 1 were employed to make the following comparative intermediates and complete formulations.

| | WEIGHT % FOR COMPARATIVE INTERMEDIATES 1 and 2 and INTERMEDIATE B | | |
|---|---|---|---|
| INGREDIENTS | 1 | 2 | B |
| Deionized water | 96.7 | 96.4 | 96.1 |
| 5% KATHON CT* | 0.20 | 0.20 | 0.20 |
| HCP** | 0.45 | 0.75 | 1.05 |
| KEMAMINE BQ-2982-B | 0.25 | 0.25 | 0.25 |
| Copolymer 845 | 1.6 | 1.6 | 1.6 |
| Fragrance 509782 | 0.80 | 0.80 | 0.80 |

5% KATHON CG represents 5 parts by weight KATHON CG (1.5% active) and 95 parts by weight deionized water.

**Hydroxyethyl cetyldimonium phosphate (BINA QAT 44C)

Comparative Intermediates 1 and 2 and Intermediate B appeared cloudy with a distinct white powdery layer on the bottom which had oil droplets dispersed in it. Intermediate A of Example 1 (a) was less cloudy than the comparative Intermediates 1 and 2 and Intermediate B, (b) had much less powdery material on the bottom and (c) had no oil droplets present on the bottom.

Comparative Formulations 1 and 2 and Formulations A and B were prepared by filling organic polymer lined tin-plate cans with 68.01 ml ofthe respective Intermediate and 81.79 ml (45.37g) of B-52 propellent. Upon shaking the pressurized containers all four formulations discharged a foam composition. Formulation A was the preferred foam composition and took less shaking for the formulation to feel solid in the can. Formulation A had the smoothest foam and appeared to be more dense than the other formulations. Comparative Formulations 1 and 2 produced foams that were not smooth in appearance when discharged from the container. Formulation B produced a smooth foam.

EXAMPLE 3

Substantially the same procedures described in Example 1 were employed to prepare the following intermediates and formulations;

| | WEIGHT PERCENT FOR INTERMEDIATES C-F | | | |
|---|---|---|---|---|
| INGREDIENTS | C | D | E | F |
| Deionized Water | 95.5 | 95.2 | 94.9 | 94.15 |
| KATHON CG, 5%* | 0.20 | 0.20 | 0.20 | 0.20 |
| HCP** | 1.65 | 1.95 | 2.25 | 3.00 |
| KEMAMINE BQ-2982-B | 0.25 | 0.25 | 0.25 | 0.25 |
| Copolymer 845 | 1.6 | 1.6 | 1.6 | 1.6 |
| Fragrance 509782 | 0.80 | 0.80 | 0.80 | 0.80 |

*5% KATHON CG represents 5 parts by weight KATHON CG (1.5% active) and 95 parts by weight deionized water.

**Hydroxyethyl cetyldimonium phosphate (BINA QAT 44C)

Intermediate C, D, E and F were evaluated for physical appearance. Intermediate C appeared cloudy with oil droplets on the bottom. Intermediate D was clearer than Intermediate C but was slightly cloudy and had some oil droplets dispersed on the bottom. Intermediate E was very slightly hazy and had no obvious separation. Intermediate F was totally clear with no separation.

Formulations C-F were prepared by filling organic polymer lined tin-plate cans with 68.01 ml of the respective Intermediate and 81.79ml (45.37g) of B-52 propellent employing the procedures and hardware described in Example 1. The foams dispensed from each Formulation were similar and had no noticeable differences. All the foams spread very smoothly and were not cheesy.

EXAMPLE 4

Intermediate A from Example 1 was admixed with B-52 propellent and filled into various organic polymer lined tin-plate cans from Continental Can Company (CCC) to make Formulation I. Substantially the same procedures and hardware described in Example 1 were employed. A prior Art Formulation was prepared employing Prior Art Intermediate and B-52 Propellent. The Prior Art Intermediate was prepared employing substantially the same procedures of Example 1 but with the following ingredients;

PRIOR ART INTERMEDIATE

| Ingredients | Weight Percent |
|---|---|
| Deionized water | 96.4 |
| KATHON CG (5%)* | 0.2 |
| VARIQUAT E228 (25%)** | 0.75 |
| KEMAMINE BQ 2982B | 0.25 |
| Copolymer 845 | 1.6 |
| Fragrance 509782 | 0.8 |

*5% KATHON CG represents 5 parts by weight KATHON CG (1.5% active) and 95 parts by weight deionized water.

**Quaternary ammonium chloride conditioning agent.

The proportions of ingredients and the specific cans and valves employed are listed below for Formulations I and the Prior Art Formulation:

FORMULATION 1

| | |
|---|---|
| Intermediate A | 93.12g |
| B-52 propellent | 111.98ml (62.12g) |
| Can | CCC 202x509 organosol lined |
| Valve | YC 900276 |

PRIOR ART FORMULATION

| | |
|---|---|
| Prior Art Intermediate | 93.12g |
| B-52 propellent | 111.98ml (62.12g) |
| Can | CCC 202x509 organosol lined |
| Valve | Precision YC 900276 |

EXAMPLE 5

A storage test was conducted with Formation I and the Prior Art Formulation and packaged in lined tin-plate cans described in Example 4 above. The cans were kept at 100°F. during the corrosion test. After nine months at 100°F one lined tin-plate can of Formulation I showed very little corrosive attack to the tin-plate can. After three months at 100°F all of the Prior Art Formulations perforated the tin-plate cans. After thirteen months at 100°F, four cans of Formulation I were examined and all were pressurized. Two of these cans had severe localized pitting while the other two had minor pitting.

In other representative operations of the present invention, various quaternary ammonium phospates, described herein, are employed as both the conditioning agent and the foaming agent in a hair conditioning foam composition whereby similar anti corrosive effects are exhibited when such hair conditioning foam compositions are packaged in organic polymer lined tin-plate cans.

**Claims**

1. A hair conditioning foam composition containing a polymeric setting and foam strengthening agent, a quaternary ammonium conditioning and foaming agent, water and a propellent, characterized by the quaternary ammonium conditioning and foaming agent being a quaternary ammonium phosphate in an amount effective to maintain the integrity of an organic polymer lining of a lined tin-plate steel can containing said composition thereby substantially preventing corrosion of the lined tin-plate steel can.

2. The composition of Claim 1, characterized in that said quaternary ammonium phosphate conditioning and foaming agent is one or a mixture of one or more compounds corresponding to the formula:

$$\left[ CH_3(CH_2)_x-\overset{R}{\underset{R^1}{N}}-CH_2\ CH_2\ OH \right]^+ \left[ H_2PO_4 \right]^-$$

wherein x represents 8 to 22, inclusive, and R and R' each independently represent an alkyl group having up to 22 carbon atoms.

3. The composition of Claim 2, characterized in that wherein the quaternary ammonium phosphate is present in said hair conditioning foam composition in an amount of from about 0.60 to about 5.0% by total weight of the hair conditioning foam composition and R and R' each represent $-CH_3$

4. The composition of Claim 3, characterized in that wherein the quaternary ammonium phosphate is present in said hair conditioning foam composition in an amount of from about 0.75 to about 3.0% by total weight of the foam composition.

5. The composition of Claim 3 or 4, characterized in that the quaternary ammonium phosphate compound is hydrosyethyl cetyldimonium phosphate.

6. A method of protecting an organic polymer lined tin-plate can from corrosion by a hair conditioning foam composition, characterized by employing as a conditioning and foaming agent for the hair conditioning foam composition a quaternary ammonium phosphate compound in an amount effective to substantially prevent corrosion of the organic polymer lined tin-plate can when the composition is stored in the tin-plate can.

7. The method of Claim 6, characterized in that the tin-plate can is lined with organosol, epon, a phenolic epoxy resin, a urea modified epoxy resin, a vinyl epoxy resin or a lacquer or an epoxy coating.

8. The method of Claim 6 or 7, characterized in that the quaternary ammonium phosphate is a compound or a mixture of compounds of the formula:

$$\left[ CH_3(CH_2)_x-\overset{R}{\underset{R^1}{N}}-CH_2\ CH_2\ OH \right]^+ \left[ H_2PO_4 \right]^-$$

wherein x represents 8 to 22, inclusive and R and R' each independently represent an alkyl group having up to 22 carbon atoms.

9. The method of Claim 8, characterized in that the quaternary ammonium phosphate compound is present in the hair conditioning foam composition in an amount of from about 0.60 to about 5.0% by total weight of the foam composition and R and R' each represent $-CH_3$.

10. The method of Claim 9, characterized in that the quaternary ammonium phosphate is hydroxyethyl cetyldimonium phosphate.